# EUROPEAN PATENT SPECIFICATION

(11) **EP 2 214 549 B1**
(45) Date of publication and mention of the grant of the patent: **08.05.2019**
(21) Application number: 08844099.5
(22) Date of filing: 30.10.2008
(51) Int. Cl.: A61B 1/00, A61B 1/005

(54) **ENDOSCOPIC OVERTUBES**
ENDOSKOP-AUSSENRÖHREN
TUBES DE RECOUVREMENT ENDOSCOPIQUE

(30) Priority: 31.10.2007 US 981134
(43) Date of publication of application: 11.08.2010
(73) Proprietor: Ethicon Endo-Surgery, Inc., Cincinnati, OH 45242 (US)
(72) Inventor: BAKOS, Gregory J., Mason Ohio 45040 (US); GHABRIAL, Ragae, Cincinnati Ohio 45241 (US); STEFANCHIK, David, Morrow Ohio 45152 (US); VAKHARIA, Omar J., Cincinnati Ohio 45230 (US)
(74) Representative: Tunstall, Christopher Stephen
(86) International application number: PCT/US2008/081717
(87) International publication number: WO 2009/058953

(56) References cited:
- EP-A- 1 477 106
- DE-A1- 3 008 120
- JP-A- 2002 369 791
- JP-A- 2003 088 494
- JP-A- 2004 065 745
- US-A- 4 770 188
- US-A- 5 405 073
- US-A- 5 976 074
- US-A1- 2007 233 040
- US-A1- 2007 233 043

## Description

### FIELD OF THE INVENTION

The present invention relates, in general, to surgical devices and, more particularly, to devices for facilitating the navigation and guidance of an endoscope within the body to accomplish various surgical and therapeutic procedures.

### BACKGROUND OF THE INVENTION

Endoscopes can be used for a variety of different diagnostic and interventional procedures. Due to their relative size and flexibility, endoscopes are well-suited for insertion through a patient's natural orifice (e.g., the patient's mouth, vagina, anus) to gain access to a particular organ or body cavity without the undesirable side effects (scarring, infection, etc.) associated with, for example, a formal laparotomy. Endoscopic surgical instruments are often preferred over traditional open surgical devices since the use of a natural orifice tends to reduce the post-operative recovery time and complications. Consequently significant development has gone into a range of endoscopic surgical instruments that are suitable for precise placement of a working end of a tool at a desired treatment site, as well as articulation and/or actuation of the working end of the device upon arrival at the treatment site. These tools can be used to engage and/or treat tissue in a number of ways to achieve a diagnostic or therapeutic effect.

Endoscopic surgery generally may require that the shaft of the device be flexible while still allowing the working end to be articulated to angularly orient the working end relative to the tissue and be actuated to treat tissue. Once in position, it may be desirable to then stiffen the device to prevent further movement thereof. For example, stiffening an endoscope can be a critical step in performing a surgical procedure in the peritoneal cavity. However, the flexible nature of an endoscope which makes it ideal for navigating a patient's internal lumen may be a disadvantage in the peritoneal cavity wherein rigidity is needed.

Over the years, a variety of different guide tubes and overtubes for endoscopes and the like have been developed for guiding and, in some instances locking, an endoscope into position. For example, U.S. Patent No. 3,470,876 to Barchilon discloses a dirigible catheter that has a lumen with continuous smooth walls between a proximal and a distal end. The distal end is guidable through 360 degrees by means of guide lines extending along the length of the catheter and which are differentially operated in pairs.

U.S. Patent No. 4,580,551 to Siegmund et al., discloses a flexible plastic tube with internal web and bore structures comprising a sequence of connected vertebra-like elements that may be used in connection with endoscopes and the like.

U.S. Patent No. 5,325,845 to Adair, discloses a steerable sheath for use with various types of optical catheters. The device includes an elongated deformable optical body that has a distal end and a proximal end. The device employs two pairs of guide wires that are attached to control knobs for pivoting the sheath in various directions.

U.S. Patent No. 5,916,147 to Boury discloses a remotely steerable catheter that has an elongate tubular member that has a proximal end and a distal end, a remotely manipulatable length, and a wall defining a lumen and first and second wires that are slidably retained by the wall. The wires are used to manipulate the length of the tubular member.

Published U.S. Patent Application No. US2006/0015009A1 to Jaffe et al. discloses a guide tube arrangement for endoscopes that is designed to be stiffened along its entire length from a relaxed configuration. The endoscope has a steerable distal portion to facilitate steering of the device through tortuous paths. In the relaxed configuration, a portion of the guide tube is able to assume the shape or curve defined by the controllable distal portion of the endoscope. Having assumed the shape of the endoscope, the guide tube may be stiffened by the surgeon to maintain that shape or curve while the endoscope is advanced distally through the tortuous path without having to place any undue pressure against the tissue walls.

US 4,770,188 describes a guide tube assembly through which an endoscope is to be inserted with the guide tube assembly and endoscope to be inserted into a body. A porous tube is formed of a material capable of being stretched axially without deformation of the tubular shape and capable of being bent without deforming the tubular shape, such as a fluorocarbon elastomer. A body of the guide tube assembly comprises separable wound sections wound around the porous tube. The separable wound sections are axially movable along the porous tube. The separable wound sections are drawn together toward a forward end of the porous tube when the porous tube is stretched axially in a configuration to form a stiff body conforming to the configuration of the porous tube to fix the guide tube assembly in this configuration. The separable wound sections are normally being separated from each other to enable the body to flex to be able to assume that configuration. The separable sections are formed by a thin metal plate spirally wound around the porous tube or by a wire spirally wound around the porous tube.

Although the previously described devices are suitable for their intended purpose, each is somewhat complex in design. Consequently a need exists for an alternative to conventional overtubes and sheaths for use with endoscopes that is less complex in design and which may be advantageously used to selectively retain a flexible endoscope in a desired orientation.

The foregoing discussion is intended only to illustrate some of the shortcomings present in the field of the invention at the time, and should not be taken as a disavowal of claim scope.

### SUMMARY

In one aspect of the invention, there is provided an overtube for use with an endoscopic surgical instrument, as disclosed in claim 1. The overtube comprises a substantially flexible helically wound continuous member that forms a series of helical coils that define a hollow passage sized to receive a portion of the endoscopic surgical instrument therethrough. The overtube has a distal end and a proximal end. A plurality of tensioning members are coupled to the overtube and extend through it to selectively draw the helical coils into abutting contact with each other to stiffen the overtube. An actuator is operably coupled to said tensioning members. The helical coils are provided with lumens for receiving the tensioning members.

In another aspect of the present invention, there is provided a surgical kit comprising an endoscope and the overtube of the previous paragraph.

These and other objects and advantages of the present invention shall be made apparent from the accompanying drawings and the description thereof.

### BRIEF DESCRIPTION OF THE FIGURES

The accompanying drawings, which are incorporated in and constitute a part of this specification, illustrate embodiments of the invention and, together with the general description of the invention given above, and the detailed description of the embodiments given below, serve to explain various principles of the present invention.
FIG. 1 is an exploded assembly view of an overtube embodiment of the present invention and an endoscope;
FIG. 2 is another view of the overtube embodiment and endoscope of FIG. 1 with the overtube installed over the elongate flexible tube of the endoscope;
FIG. 3 is another view of the overtube and endoscope depicted in FIG. 2 wherein the overtube and flexible endoscope tube have been articulated;
FIG. 4 is a perspective view of a portion of another overtube embodiment of the present invention;
FIG. 5 is an exploded assembly view of the overtube embodiment of FIG. 4 and an endoscope;
FIG. 6 is another view of the overtube embodiment and endoscope of FIG. 5 with the overtube installed over the elongate flexible tube of the endoscope;
FIG. 7 is another view of the overtube and endoscope depicted in FIGS. 5 and 6 wherein the overtube and flexible endoscope tube have been articulated;
FIG. 8 is a perspective view of a portion of another overtube embodiment of the present invention;
FIG. 9 is another perspective view of the overtube embodiment of FIG.8 with tension members extending therethrough;
FIG. 10 is an isometric view of one embodiment of an endoscopic device having a control mechanism with an overtube embodiment of the present invention installed thereon;
FIG. 11 is a side view of an actuator of the device of FIG. 10;
FIG. 12 is an isometric view of the actuator of FIG. 11 showing a trigger coupled thereto;
FIG. 13 is an exploded view of the actuator of FIG. 10;
FIG. 14 is another view of the actuator of FIG. 10;
FIG. 15 is another view of the actuator of FIG. 10;
FIG. 16 is an exploded view of a driver and a spreader plate of the actuator of FIG. 10;
FIG. 17 is another isometric view of the device and overtube of FIG. 10, with the overtube in a stiffened condition;
FIG. 18 is a perspective view of a portion of another overtube embodiment of the present invention;
FIG. 19 is another perspective view of the overtube of FIG. 18 in a stiffened condition;
FIG. 20 is a perspective view of a portion of another overtube embodiment of the present invention;
FIG. 21 is a perspective view of the portion of the overtube of FIG. 20 installed on a portion of an elongate endoscope tube;
FIG. 22 is a perspective view of a portion of another overtube embodiment of the present invention;
FIG. 23 is a perspective view of a portion of another embodiment of the present invention;
FIG. 24 is a perspective view of a device of the present invention attached to another overtube embodiment of the present invention;
FIG. 25 is a portion of the overtube depicted in Figure 24 in cross-section;
FIG. 26 is a portion of the overtube of Figures 24 and 25; and
FIG. 27 is a perspective view of a coil of the overtube of Figures 24-26.

### DETAILED DESCRIPTION

Certain exemplary embodiments will now be described to provide an overall understanding of the principles of the structure, function, manufacture, and use of the devices and methods disclosed herein. One or more examples of these embodiments are illustrated in the accompanying drawings. Those of ordinary skill in the art will understand that the devices and methods specifically described herein and illustrated in the accompanying drawings are nonlimiting exemplary embodiments and that the scope of the various embodiments of the present invention is defined solely by the claims. The features illustrated or described in connection with one exemplary embodiment may be combined with the features of other embodiments. Such modifications and variations are intended to be included within the scope of the present invention.

The present invention generally relates to various devices and overtube arrangements for use in connection with surgical instruments such as, for example, endoscopes for selectively retaining a flexible endoscope in a desired orientation.

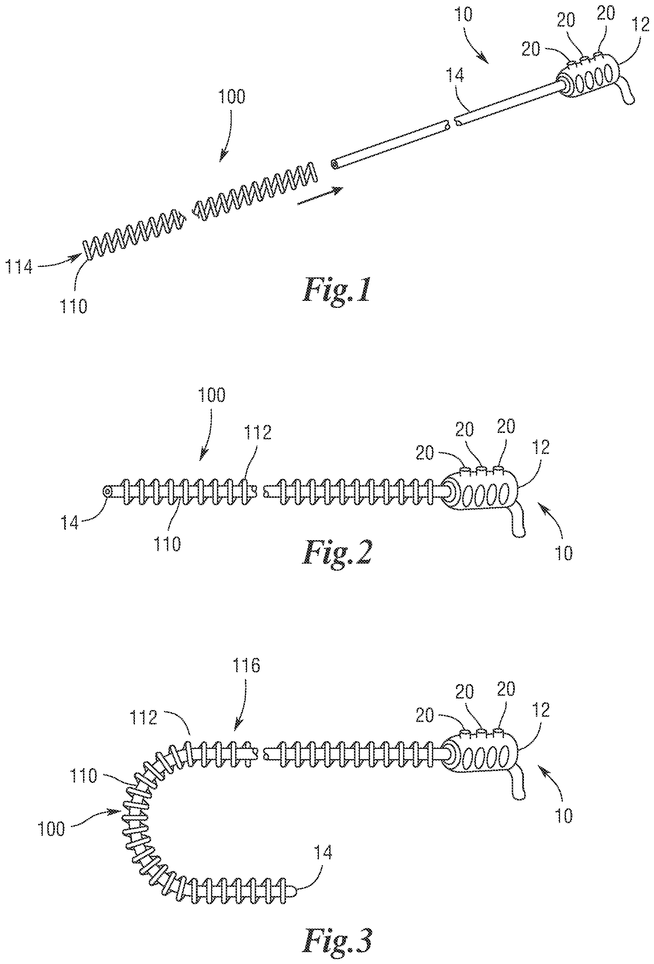

More specifically and with reference to FIGS. 1-3, there is shown an endoscope 10 with which an overtube 100 of the present invention may be used. By way of example, the endoscope 10 may comprise a handle 12 that is attached to an elongated flexible tube 14 that may support a camera (not shown) therein. The elongated flexible tube 14 may further have one or more working channels (not shown) therein that may function as lumens for receiving additional surgical tools therethrough. The working channels may be used to apply suction, pressurized air, fluid, etc. to an area within the body. The handle 12 may have a series of control buttons 20 or the like thereon that serve to control various instruments within the flexible tube 14 or remotely mounted suction and/or aspiration units (not shown).

It will be appreciated that the terms "proximal" and "distal" are used herein with reference to a clinician manipulating the handle portion 12 of the endoscope 10 . The term "proximal" referring to the portion closest to the clinician and the term "distal" referring to the portion located away from the clinician. It will be further appreciated that for convenience and clarity, spatial terms such as "vertical", "horizontal", "up" and "down" may be used herein with respect to the drawings. However, surgical instruments are used in many orientations and positions, and these terms are not intended to be limiting and absolute.

FIGS. 1-4 illustrate, in general form, one exemplary overtube 100 of the present invention that can be used in connection with a flexible endoscope 10. As can be seen in those Figures, the overtube 100 comprises a helically wound continuous member or rod 110 that serves to form a series of helical coils 112 that define a passage 114 for receiving the flexible endoscope tube 14 therethrough. In various embodiments, for example, the continuous member may be fabricated from Polytetrafluoroethylene, Nylon, Polyethylene, Polyetheretherketone, etc. The outer surface of 116 of the member or rod 110 may be substantially smooth to avoid, for example, trauma to a gastrointestinal lumen. In one embodiment, for example, once the elongate tube 14 of the endoscope 10 has been moved into a desired area, the helical coils 112 can simply be pushed together by applying an axial force to the proximal end of the overtube 100 to cause the coils to collectively form a substantially fluid-tight passage for supporting the elongated tube 14 or other instruments therein.

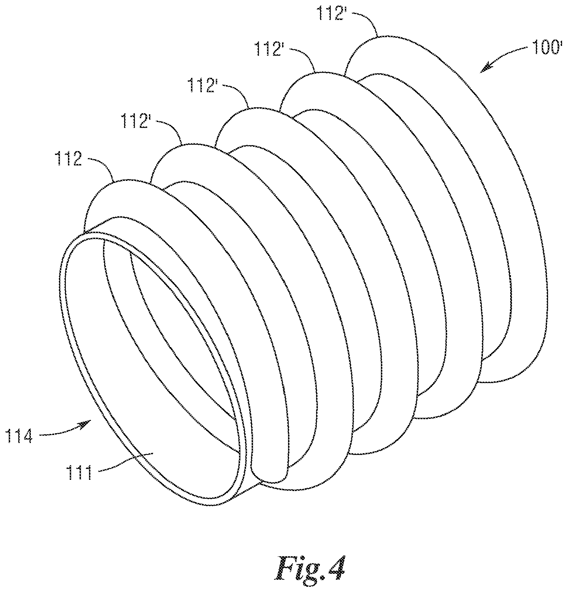

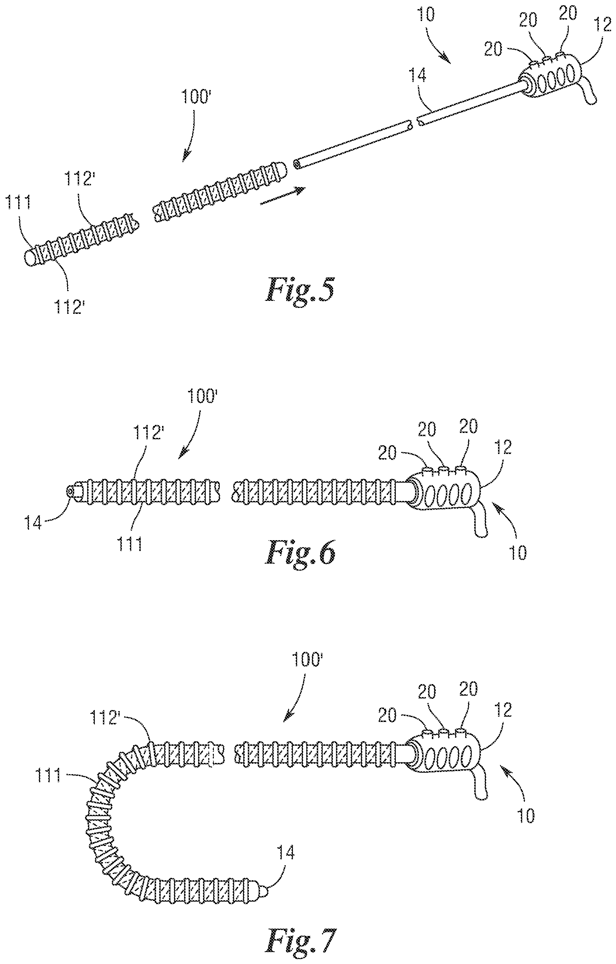

In other embodiments, the overtube 100' may be formed from a series of helical coils 112' that are integrally formed (molded) around or otherwise attached to a flexible tubular member 111 that serves to form a substantially fluid-tight passage 114' for supporting the elongated tube 14 of the endoscope 10 therein. See FIGS. 4-7. The overtube 100' may be fabricated from, for example, Polytetrafluoroethylene, Nylon, Polyethylene, Polyetheretherketone or a combination thereof, such that the tubular member 111' thereof may sufficiently collapse into an abutting relationship so that the adjacent coils 112' may be sufficiently pushed together to stiffen the overtube 100'.

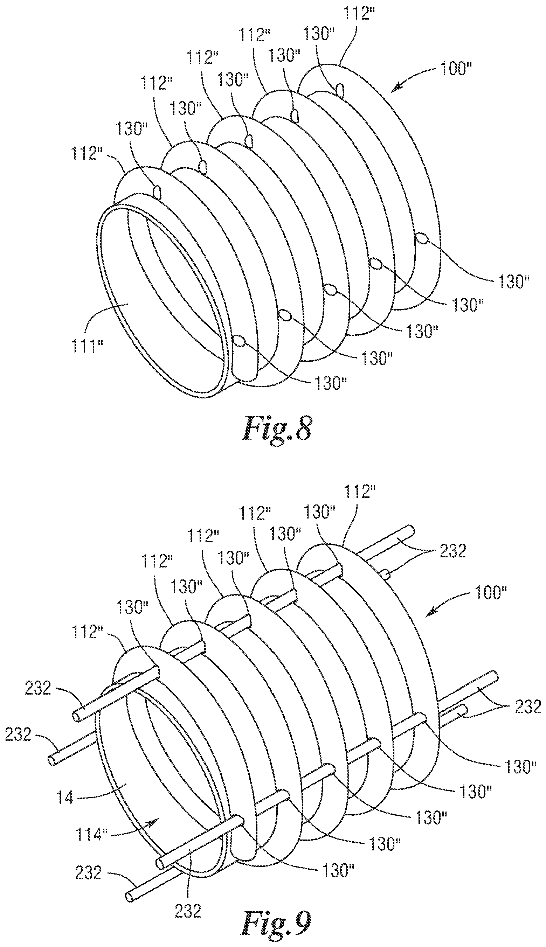

FIGS. 8 and 9 illustrate an alternative overtube embodiment 100" of the present invention that is configured to receive tension elements (not shown in FIG. 8) to facilitate pulling the adjacent coils 112" together. In this embodiment, for example, the coils 112" may be substantially identical to coils 112' described above, except that the coils 112" may be provided with lumens or passages 130" for receiving at least one tensioning element 232 therethrough. The overtube 100" may be fabricated from, for example, Polytetrafluoroethylene, Nylon, Polyethylene, Polyetheretherketone, such that the tubular portion 111" may sufficiently collapse into an abutting relationship so that the adjacent coils 112" may be pulled adjacent to each other as will be further discussed below.

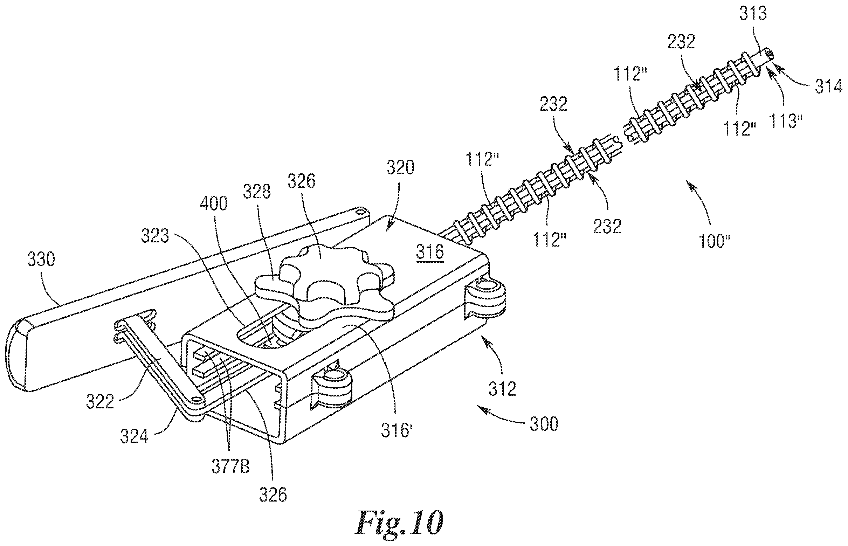

As can be seen in FIGS. 10-17, the overtube 100" may be used in connection with an endoscopic device 300 of the type disclosed in commonly owned U.S. Patent Application Serial No. 11/762,855, entitled CONTROL MECHANISM FOR FLEXIBLE ENDOSCOPIC DEVICE AND METHOD OF USE, filed June 14, 2007 to James T. Spivey and Omar J. Vakharia. As can be seen in FIG. 10, the device 300 may include a handle 312 that has and elongated flexible tube 314 protruding therefrom that may further have one or more working channels (not shown) therethrough. The overtube 100" is sized to be received over the elongated tube 314 and has a distal end 113" that corresponds to a distal end 313 of the elongate flexible tube 314. The elongated tube 314 may further have a proximal end 315 that is coupled to the handle portion 312 that is adapted to remain external to the patient's body.

In various embodiments, the tensioning elements 232 extend from an actuator 320 and the distal end 213 of the overtube 100" to facilitate tensioning thereof to allow for the controlled articulation of the distal end 313 of the elongate tube 314 and ultimately the locking or rigidifying of the overtube 100" if so desired. The actuator 320 can be disposed within the handle 312, and a trigger 330 can be coupled to the actuator 320. The handle 312 can also include an outer housing 316 that houses at least a portion of the actuator 320 and that has the trigger 330 formed thereon or mated thereto. The tensioning element or elements 232 are capable of coupling the actuator 320 to the distal end 113" of the overtube 100", and further capable of being tensioned to articulate (or otherwise effect action of) the distal end 113" of the overtube 100" and ultimately to rigidify the overtube 100". For example, the tensioning element or elements 232 can be a wire, a cable, a fiber, etc. In various embodiments, the tensioning elements 232 may be substantially U-shaped or have a linear configuration as taught in the aforementioned commonly owned U.S. Patent Application Serial No. 11/762,855.

In order to allow movement of the tensioning elements 232 to articulate or rigidify the overtube 100", the tensioning elements 232 may be slidably supported in the overtube 100". While various mating techniques can be used to allow slidable movement of the tensioning elements 232, in one embodiment the coils 112" of the overtube 100" can include a number of lumens 130" sized to allow the tensioning element(s) 232 to slidably extend therethrough. See FIG. 8. The distal end of each tensioning element 232 can include a ball-shaped element (not shown) having a diameter that is larger than a diameter of the lumen 130". The ball-shaped element will thus prevent the distal ends of the tensioning elements 232 from being pulled into the lumens 230. Those skilled in the art will appreciate that various other mechanisms can be used to mate the ends of the tensioning elements 232 to the distal-most end of the overtube 100". The proximal U-shaped end of each tensioning element 232 can be positioned within a handle housing 316, and thus the housing 316 can include four openings (not shown) formed therein and axially aligned with the lumens 130" in the overtube coils 112" to allow the tensioning elements 232 to extend into the housing 316. Additionally, one or more lumens in the elongate shaft 314 can also be axially aligned with a working channel lumen of the housing 316 which extends to a working channel port capable of introducing various tools through the shaft 314 to the treatment site.

The device 300 may include an actuator 320 which can be manipulated between first and second positions so as to engage and disengage the tensioning element(s) 232. In the first position (i.e., the disengaged state), the tensioning elements 232 can move freely relative to the actuator 320 to allow the overtube 100" to flex freely enroute to the treatment site. The actuator 320 can also be configured to provide slack to the tensioning elements 232 in the first position, as will be discussed in more detail below. In the second position (i.e., the engaged state), the actuator 320 can engage and apply tension to the tensioning elements 232 to lock the coils 112" of the overtube 100" together.

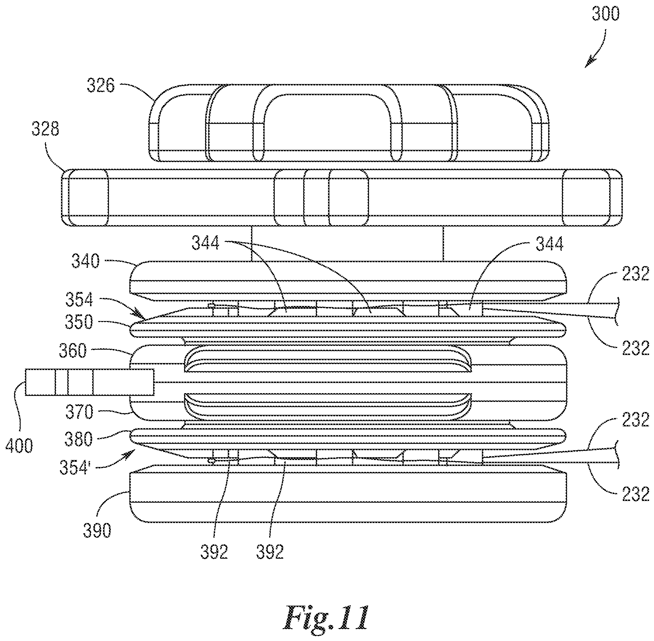

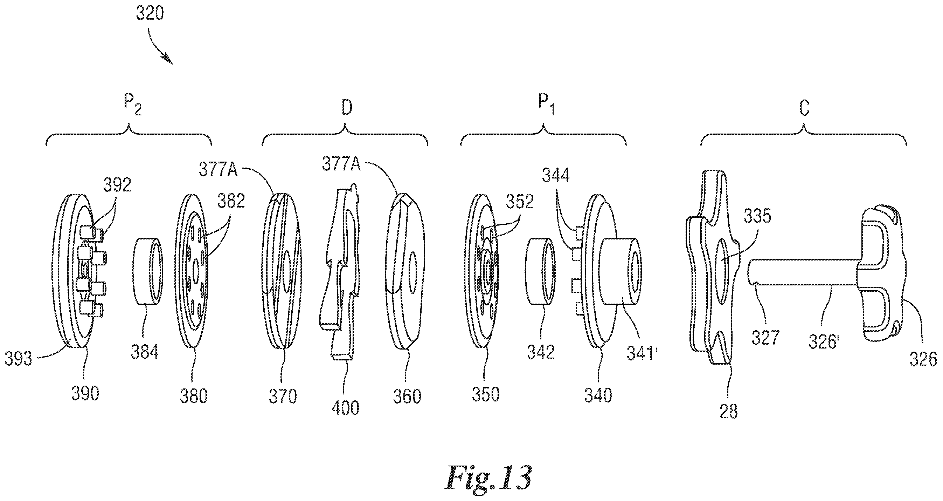

FIGS. 11-16 illustrate one embodiment of an exemplary actuator 320. As best shown in FIG. 13, the actuator 320 can be configured as a spool or spool-like element having a first spool member P₁ configured to receive a first tensioning element 232, and a second spool member P₂ configured to receive a second tensioning element 232. The actuator 320 can also include a drive mechanism D adapted to move the spool members P₁, P₂ between open and closed positions for releasing and engaging the tensioning elements 232, and a control mechanism C for controlling the spool members P₁, P₂ when the spool members P₁, P₂ are in the engaged positions so as to selectively move the tensioning elements 29, 31. A person skilled in the art will appreciate that the actuator 320 can include any number of spool members for receiving a corresponding number of tensioning elements 232.

Each spool member P₁, P₂ can have a variety of configurations, but in an exemplary embodiment each spool member P₁, P₂ include first and second opposed plates 340, 350, 380, 390 that are adapted to receive the tensioning elements 232 therebetween. Each plate 340, 350, 380, 390 can have various shapes and/or dimensions. As shown in FIG. 13, the plates 340, 350, 380, 390 are substantially circular. As for dimensions, those skilled in the art will appreciate that plates 340, 350, 380, 390 having a wide range of diameters and/or thickness are within the spirit and scope of the present invention. Additionally, the plates 340, 350, 380, 390 can be configured in various ways so as to facilitate receipt of the tensioning elements 232 therebetween. In the illustrated embodiment, each pair of plates 340, 350, 380, 390 includes one stationary plate 340, 390 having a plurality of posts 344, 392 extending therefrom, and an opposed movable plate 350, 380 having a series of corresponding openings 352, 382 adapted to receive the posts 344, 392. The posts 344, 392 can be positioned in a circular pattern around an outer portion of each plate 340, 390 for receiving the U-shaped end of the tensioning element 232 therearound, and the openings 352, 382 can be similarly disposed in a circular pattern around an outer portion of each stationary movable plate 350, 380. While not shown, the plates 340, 350, 380, 390 can also include additional features to prevent a slackened tensioning element 232 from becoming disengaged with the pair of opposed plates 340, 350, 380, 390 (i.e., falling out from between the plates 340, 350, 380, 390). For example, an outer-most edge of the opposed plates 340, 350, 830, 390 can include a second set of posts or a housing (not shown) and the tensioning elements 232 can extend between the two sets of posts (or the posts and the housing). Those skilled in the art will appreciate that various other techniques can be used to retain the tensioning elements 232 between the plates 340, 350, 380, 390.

The plates 340, 350, 380, 390 can also include other features to facilitate receipt of the tensioning elements 232 therebetween. For example, as best shown in FIG. 11, an outer perimeter of at least one of plates of each pair (e.g., the movable plates 350, 380) can include a ramped portion 354, 354' which facilitates receipt of the tensioning elements 232 therebetween. Additionally, the surface of any of the plates 340, 350, 380, 390 which contacts the tensioning elements 232 can include various features, such as surface features, a roughening, a chemical treatment, etc., capable of assisting in securing the tensioning elements 232 to the plates 340, 350, 380, 390. Those skilled in the art will appreciate that any techniques can be utilized to better contact and/or secure the tensioning elements 232 between the opposed plates 340, 350, 380, 390.

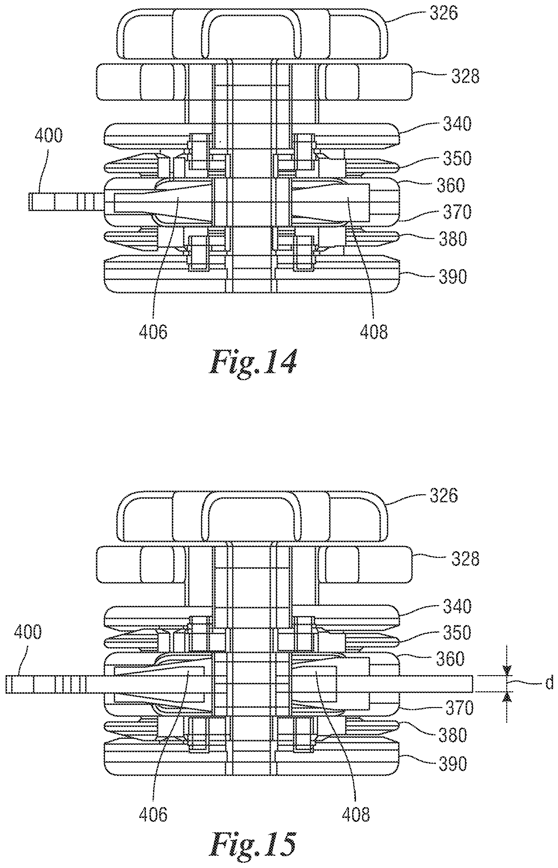

The plates 340, 350, 380, 390 can also be movably coupled to one another to allow the plates 340, 350, 380, 390 to engage and disengage the tensioning elements 232. In an exemplary embodiment, the plates 340, 350, 380, 390 are movable between a biased, open position, shown in FIG. 15, in which the plates 340, 350, 380, 390 are spaced a distance apart from one another to allow free slidable movement of the tensioning elements 232 therebetween, and a closed position, shown in FIG. 14, in which the plates 340, 350, 380, 390 are moved together to engage the tensioning elements 232 therebetween so as to prevent free movement of the tensioning elements 232. Those skilled in the art will appreciate that each pair of plates 340, 350, 380, 390 can be moved from the biased, open position to the closed position using various techniques. In the illustrated embodiment, a biasing element, such as a wave spring 342, 384 (FIG. 13), can be disposed between each pair of plates 340, 350, 380, 390 so as to bias each pair 340, 350, 380, 390 apart from one another to an initial open position. In response to an actuation force greater than the force supplied by the biasing element 342, 384, the movable plate 350, 380 of each pair of plates 340, 350, 380, 390 can slide along the respective plurality of posts 344, 392 towards the opposed stationary plate 340, 390 to engage the tensioning element 232 disposed therebetween. In other embodiments, the pairs of plates 340, 350, 380, 390 can be mounted such that all four plate move in response to the actuation force.

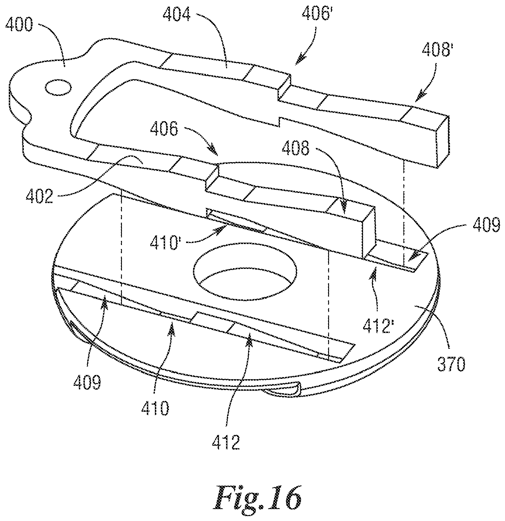

As indicated above, the actuator 320 can also include a drive mechanism D adapted to move the first and second spool members P₁, P₂ between the disengaged (open) and engaged (closed) positions. In general, the drive mechanism D can be any mechanism configured to apply a force to one or both plates of each pair of opposed plates 340, 350, 380, 390 to overcome the biasing force. In an exemplary embodiment, the drive mechanism D is movably disposed between the movables plates 350, 380 of each pair of plates 340, 350, 380, 390 to push the movable plates 350, 380 toward the stationary plates 380, 390 and into the closed position. In the illustrated embodiment, the drive mechanism D includes a set of spreader plates 360, 370 disposed between the pairs of opposed plates 340, 350, 380, 390, and a wedge 400 disposed between the spreader plates 360, 370. Referring to FIGS. 11-17, a first spreader plate 360 can be positioned immediately below the first movable plate 350 and a second spreader plate 370 can be positioned immediately above the second movable plate 380. The spreader plates 360, 370 can be adapted so as to slidably receive the wedge 400 therebetween. FIG. 16 illustrates spreader plate 370, however spreader plate 360 can have a similar configuration. As shown, the spreader plate 370 can include a first cavity 409 and a second cavity 409' adapted to slidable seat a first extension 402 and a second extension 404 of the wedge 400. Additionally, the first extension 402 of the wedge 400 can include first and second ramped elements 406, 408 formed thereon, and the second extension 404 of the wedge 400 can also include first and second ramped elements 406', 408' formed thereon. The first cavity 409 of the spreader plate 470 can include a first ramped portion 410 and a second ramped portion 412, and the second cavity 409' can also include first and second ramped portions 410', 412'. As clearly illustrated in FIG. 15, as the wedge 400 is pulled in a proximal direction, each ramped element 406, 408 is pulled into the corresponding ramped portion 410, 412 thereby forcing the spreader plates 360, 370 a distance d apart from one another. The movable plates 350, 380 will move the same distance towards their respective opposed stationary plates 340, 390. As a result, the tensioning elements 232 extending between the plates 340, 350, 380, 390 will be engaged by an interference or compression fit between the two plates 340, 350, 380, 390. One skilled in the art will appreciate that any number and/or orientation of ramps, cavities, drivers, spreader plates, etc. are within the spirit and scope of the present invention. As illustrated in the exemplary embodiment discussed above, strategic placement of the ramped elements and/or cavities can provide added stability to the control mechanism (i.e., placing the ramped elements symmetrically about a central position of the spreader plates 360, 370.) However, any such positioning can be utilized (e.g., a single ramp element placed in communication with any location of the spreader plate(s)). Additionally, in other embodiments, the movable plates 350, 380 themselves can be adapted to receive the ramped elements thereby eliminating the spreader plates.

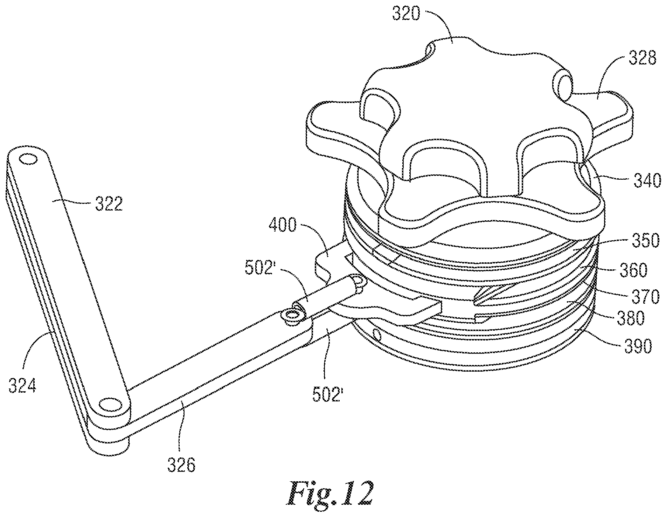

In order to move the wedge 400 relative to the spreader plates 360, 370, the actuator 320 can further include a trigger 330 adapted to actuate the drive mechanism. In an exemplary embodiment, the trigger 330 is adapted to supply a proximal force to the driver D thereby pulling the wedge 400 to pull the ramped elements 406, 408 into the corresponding ramped portions of the cavities of the spreader plates 360, 370 to drive the spreader plates 360, 370 apart from one another. Various types of triggers can be utilized. FIG. 12 illustrates two linking elements 502, 502', in the form of springs, that are coupled at a first end to the wedge 400 and at a second end to a first linkage arm 326. The other end of the first linkage arm 326 is coupled to first and second linkage elements 322, 324 which in turn are coupled to a trigger 330. One end of the trigger 330 can be pivotally mated to the housing 316 to allow the trigger 330 to pivot toward and away from the housing 316 to move between closed and open positions, respectively. The trigger 330 can be biased to an open position, shown in FIG. 10, due to the wave springs 342, 384 that bias the plates 340, 350, 380, 390 to the open position. In use, as the trigger 330 is compressed toward the housing 316 (e.g., via a user supplied force), the second linkage arms 322, 324 can pull on the first linkage arm 326 in a proximal direction, thereby pulling the wedge 400 in a proximal direction to force the spreader plates 360, 370 apart. When the force is removed from the external trigger 330 (e.g., releasing the trigger 330), the spreader plates 360, 370 are allowed to return to their original position which thereby returns each pair of opposed plates 340, 350, 380, 390 to their respective biased, open positions. Those skilled in the art will appreciate that various other components and/or linkages are within the spirit and scope of the present invention.

The trigger 330 can also be adapted to provide slack to the tensioning elements 232 when the actuator 320 is in the first position, and to supply a desired tension to (or to remove slack from) the tensioning elements 232 prior to engaging the elements 232 between respective opposed plates 340, 350, 380, 390. For example, when the trigger 330 is in the initial position, the entire actuator 320 is free to slide distally within the housing 316 to provide slack to the tensioning elements 232, and when the trigger 330 is actuated, an initial force can slide the entire actuator 320 in a proximal direction within the housing 316 such that the tensioning elements 232, are tensioned around the respective pair of plates (i.e., the slack is removed), and an additional force can pull only the wedge 400 in the proximal direction so as to lock the tensioning elements 232 between the respective pairs of plates 340, 350, 380, 390. More specifically, looking at FIG. 10, a set of grooves 377B can be incorporated into an inner portion of the housing 316 and they can be configured to receive a corresponding portion 377A (FIG. 13) of the spreader plates 360, 370 to allow the actuator 320 to slide within the housing 316. The actuator 320 can be biased to a distal portion of the housing 320. This can result from the wave springs 342, 384 biasing the spreader plates 360, 370 toward one another. Since movement of the wedge 400 within the housing 316 is limited by the trigger 330, as the spreader plates 360, 370 are moved together by the wave springs 342, 384, the entire actuator 320 (except the wedge 400) will move within the housing relative to the wedge 400. Thus, as the trigger 330 is actuated, the entire actuator 320 slides proximally within the housing 316 until the tension supplied by the tensioning element(s) 232 reaches an amount which prevents the actuator 320 from sliding any further in the proximal direction. While the actuator 320 is prevented from any further proximal movement, the wedge 400 can continue to move in the proximal direction thereby locking the tensioning element(s) 232 therebetween. Those skilled in the art will appreciate that various other mechanisms and/or configurations can be utilized so as to allow the assembly to uniformly slide within the housing 320. Moreover, while this example allows for a single trigger 330 to both tension the tensioning element(s) and secure the elements between respective pairs of opposed plates, those skilled in the art will appreciate that in other embodiments distinct levers and/or trigger assemblies can be utilized to perform these functions.

As mentioned above, the device 300 can also include a control mechanism C configured to control axial movement of the tensioning elements 232 to thereby articulate or rigidify the overtube 200. While various control mechanisms can be used, in one exemplary embodiment, as shown in FIG. 8, the top 316' of the housing 316 can include an opening 323 having first and second control members 326, 328 coupled to the actuator 320. In particular, the first and second control members 326, 328 can be coupled to the first and second pair of opposed plates 340, 350, 380, 390, respectively, to allow the control members 326,3 28 to apply a rotational force to the plates 340, 350, 380, 390 which in turn will cause axial translations of the tensioning elements 232 engaged therebetween. Each control member 326, 328 can have a variety of configurations, but in one exemplary embodiment each control member 326, 328 is in the form of a rotational knob. As shown in FIG. 13, the first control member 326 can have a shaft 326' extending downward and configured to pass through central openings in each of the various components discussed above and illustrated in FIG. 13. The distal end of the shaft 326' can include a pin-hole 327 that can be aligned with a corresponding pin-hole 393 of the bottom-most plate 390 so as to allow a pin (not shown) to be placed through the corresponding pin-holes 327, 393 to thereby mechanically couple the control member 326 to the second pair of opposed plates 380, 390. The second control member 328 can be rotatably disposed around the shaft 326' of the first control member 326, and it can include a similar pin-hole 335 adapted to be aligned with a pin-hole (not shown) formed in a cylindrical member 341' extending from the top stationary plate 340 and extending into the central opening in the second control member 328. Thus, insertion of a second pin (not shown) through pin-holes will fixedly mate the second control member 328 to the first pair of opposed plates 340, 350. As will be apparent to those skilled in the art, any number of control members can be coupled to any number of corresponding pairs of opposed plates using various mating techniques known in the art.

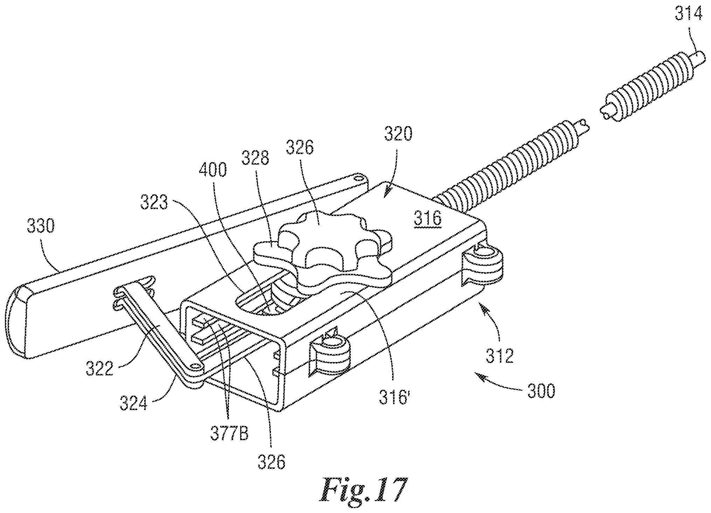

As tension is applied to the tensioning elements 232, the tensioning elements 232 draw the distal end of the overtube 100" proximally causing the coils to be drawn into contact with each other to stiffen the overtube 100" and thereby lock the flexible elongate tube 14 of the endoscope 10 in position. See FIG. 17.

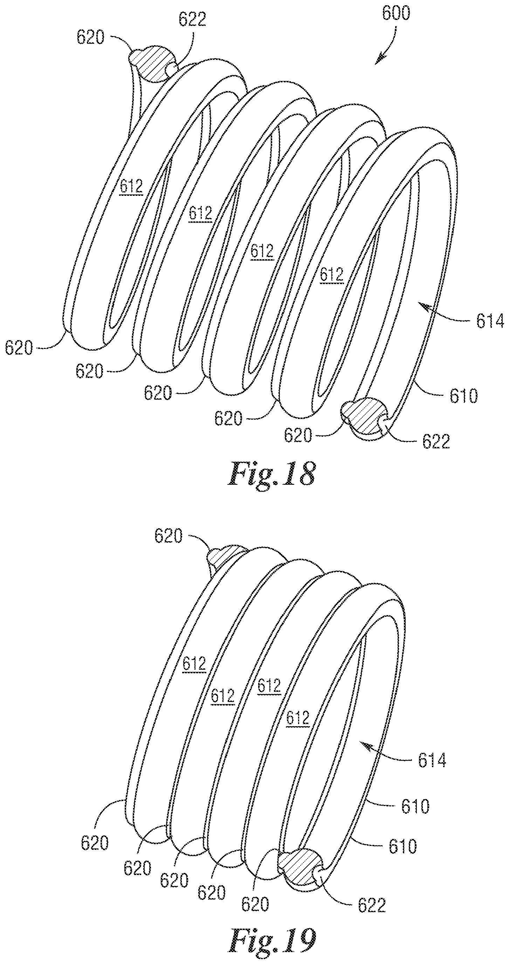

FIGS. 18 and 19 illustrate another overtube 600 of the present invention that may be employed with, for example, an endoscope 10 of the type and construction described above. As can be seen in Fig. 18, the overtube 600 may be formed from a helically wound member or rod 610 that forms a series of helical coils 612 that define a passageway 614. The rod 610 may be fabricated from, for example, Polytetrafluoroethylene, Nylon, Polyethylene, Polyetheretherketone. In these embodiments, however, the rod 610 may be advantageously configured with a protrusion 620 on one side of the rod 610 and a pocket 622 on the other side of the rod 610. The pocket 622 may be sized relative to the protrusion 620 to frictionally receive the protrusion 620 of an adjacent coil 612 therein. Such arrangement enables the adjacent coils 612 to be selectively locked together to lock the stiffened overtube 600 in position. See FIG.19. Other interlocking shapes could also be employed.

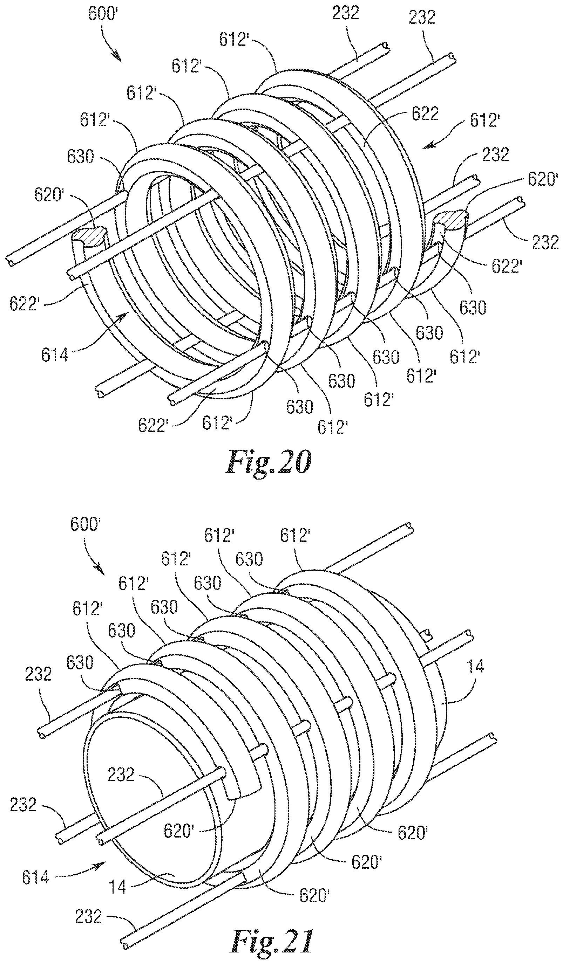

FIGS. 20 and 21 illustrate another overtube embodiment 600' that is substantially identical to overtube embodiment 600 except that the coils 612' have lumens 630 for receiving the tension members 632 therethrough for drawing the coils 612' together to rigidify or stiffen the tube 600' in the manners described above, using, for example, the device 300 described above. Thus, when the tension members 232 are pulled proximally, the distal end of the overtube 600' is also pulled proximally causing the protrusions 620' of each coil 612' to be frictionally received in the pocket 622' of an adjacent coil 612' to lock the coils 612' together.

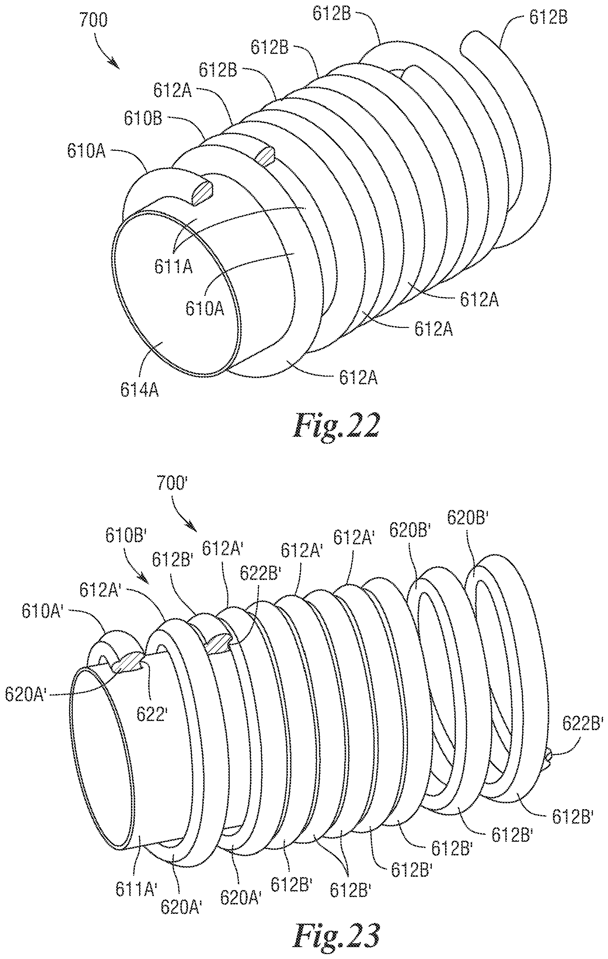

FIG. 22 illustrates another overtube assembly 700 of the present invention wherein a first helically wound member 610A is attached to or molded into a tubular member 611A to form an overtube structure 600A consisting of spaced helical coils 612A. The member 610A and the tubular member 611A may be fabricated from Polytetrafluoroethylene, Nylon, Polyethylene, Polyetheretherketone or a combination thereof. The tubular member 611A defines a fluid-tight passage 614A through which the elongated tube 14 of an endoscope 10 may pass. This embodiment further includes a second helically wound member 610B that is screwed onto the tubular member 611A between the spaced coils 612A to form coils 612B as shown The second helically wound member serves to stiffen the portions of the overtube assembly 700 upon which its is installed.

FIG. 23 illustrates an alternative overtube embodiment 700' of the present invention wherein a first helically wound member 610A' is attached to or molded into a tubular member 611A' to form an overtube structure 600A' consisting of spaced helical coils 612A' wherein the first helically wound member 610A' has a protrusion 620A' formed on one side thereof and a pocket 622A' formed on the other side thereof. Likewise, the second helically wound member 610B' has a protrusion 620B' formed on one side thereof sized to be received in the pocket 622A' of the first helically wound member 610A' and a pocket 622B formed on the other side thereof sized to receive the protrusion 620A' of the first helically wound member 610A' as the coils 612B' of the second helically wound member 610B' is screwed thereon to stiffen the over tube 700'. Those of ordinary skill in the art will appreciate that, if it is desired to only stiffen a portion of the overtube structure 700', then a segment of the second helically wound member 610B' may be wound onto or otherwise installed onto the section of the overtube 700' that is to be stiffened. If the entire overtube 700' is to be stiffened, the second helically wound member 610B' would be provided with a sufficient length such that when installed it extends substantially the entire length of the overtube 700'.

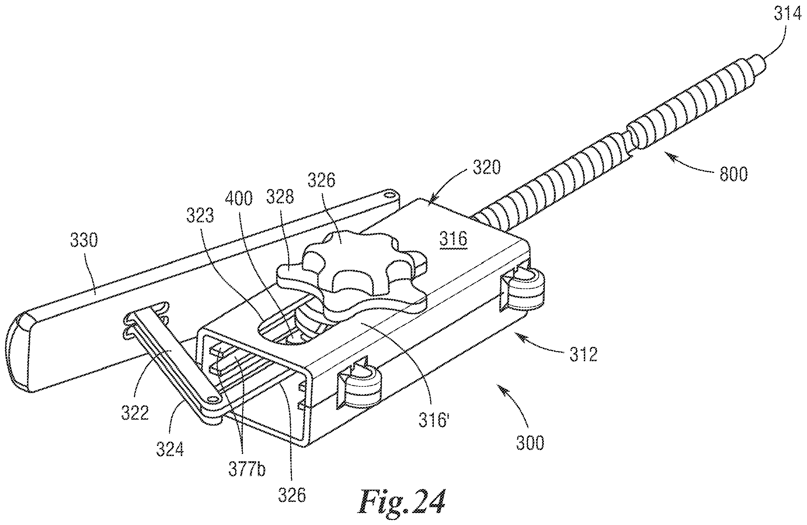

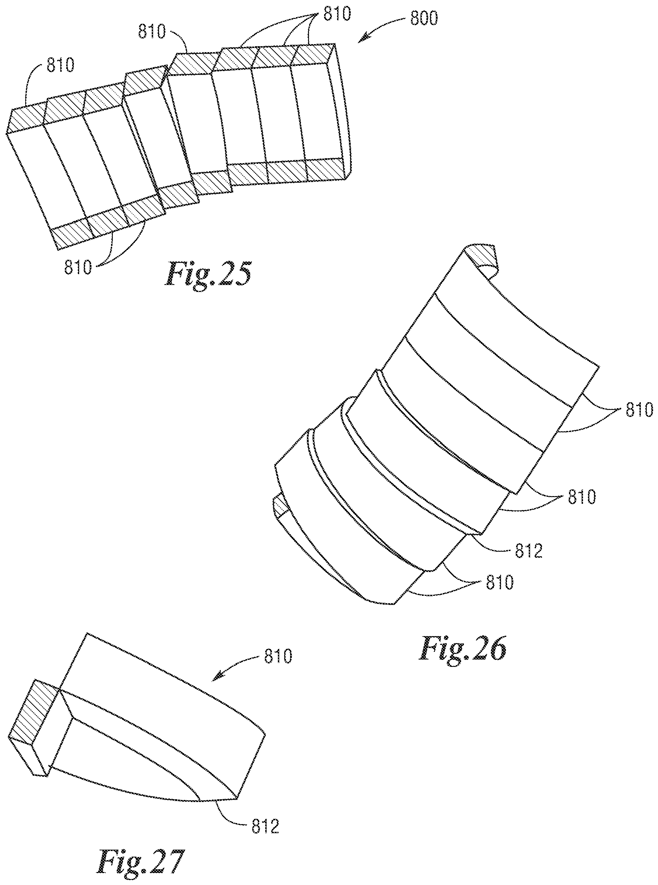

FIGS. 24-27 depict another overtube embodiment 800 of the present invention that may be, for example, employed with the endoscopic device 300 described hereinabove. The overtube embodiment 800 may comprise a substantially hollow coil pipe member, that contains a plurality of coils 810 that have a substantially parallelogram-shaped cross-section that can be compressed together such that the overtube 800 can be locked in a particular orientation. In one embodiment, the overtube 800 may be fabricated from metal material. As illustrated in FIG. 27, each coil 810 may be provided with at least one coil mating contact surface 812 that can be somewhat spherical or arcuate in shape to reduce friction and assist in managing surface loads between the coils. To facilitate stiffening of the overtube 800, it will be appreciated that the coils 812 may have lumens therethrough (not shown) to receive the tensioning elements 232 of the device 300 in the manner described above.

Other overtube embodiments of the present invention may, for example, have an accessory attached to the distal end of the overtube. For example, overtube 800 may have a grasper (not shown) attached to its distal end. The tissue could be grasped in the jaws of the grasper at the beginning of the actuation stroke. The end of the actuation stroke may compress the overtube 800, in turn locking or stiffening the tube 800. Such arrangement would enable the clinician to manipulate tissue as the rigid overtube 800 would facilitate lifting, pushing an pulling of the tissue more effective.

While the present invention has been illustrated by description of several embodiments and while the illustrative embodiments have been described in considerable detail, it is not the intention of the applicant to restrict or in any way limit the scope of the appended claims to such detail. Additional advantages and modifications may readily appear to those skilled in the art. Those of ordinary skill in the art will readily appreciate the different advantages provided by these various embodiments. For example, the various overtube embodiments disclosed herein may be advantageously used in applications wherein the clinician requires a passage that has a relatively tight bend. The unique and novel arrangements of the various embodiments of the present invention may be used in such applications without the danger of the passage collapsing or kinking. Various embodiments of the present invention may also be used to steer the endoscope in a preferred direction and then the overtube may be stiffened to lock the endoscope into a particular orientation.

While several embodiments of the invention have been described, it should be apparent, however, that various modifications, alterations and adaptations to those embodiments may occur to persons skilled in the art with the attainment of some or all of the advantages of the invention. For example, according to various embodiments, a single component may be replaced by multiple components, and multiple components may be replaced by a single component, to perform a given function or functions.

The devices disclosed herein can be designed to be disposed of after a single use, or they can be designed to be used multiple times. In either case, however, the device can be reconditioned for reuse after at least one use. Reconditioning can include an combination of the steps of disassembly of the device, followed by cleaning or replacement of particular pieces, and subsequent reassembly. In particular, the device can be disassembled, and any number of particular pieces or parts of the device can be selectively replaced or removed in any combination. Upon cleaning and/or replacement of particular parts, the device can be reassembled for subsequent use either at a reconditioning facility, or by a surgical team immediately prior to a surgical procedure. Those of ordinary skill in the art will appreciate that the reconditioning of a device can utilize a variety of different techniques for disassembly, cleaning/replacement, and reassembly. Use of such techniques, and the resulting reconditioned device, are all within the scope of the present application.

Preferably, the invention described herein will be processed before surgery. First a new or used instrument is obtained and, if necessary, cleaned. The instrument can then be sterilized. In one sterilization technique, the instrument is placed in a closed and sealed container, such as a plastic or TYVEK® bag. The container and instrument are then placed in a field of radiation that can penetrate the container, such as gamma radiation, x-rays, or higher energy electrons. The radiation kills bacteria on the instrument and in the container. The sterilized instrument can then be stored in the sterile container. The sealed container keeps the instrument sterile until it is opened in the medical facility.

## Claims

1. An overtube (100) for use with an endoscopic surgical instrument (10), said overtube comprising:
a substantially flexible helically wound continuous member (110) forming a series of helical coils (112) that define a hollow passage sized to receive a portion (14) of the endoscopic surgical instrument therethrough, said overtube having a distal end and a proximal end; and
at least one stiffening element coupled to said overtube to selectively draw said helical coils into abutting contact with each other to stiffen said overtube, wherein said at least one stiffening element comprises:
a plurality of tensioning members (232) coupled to said over tube and extending therethrough; and
an actuator (320) operably coupled to said plurality of tensioning members;
**characterized in that**, the helical coils are provided with lumens (130) for receiving said plurality of tensioning members therethrough.

2. The overtube of claim 1 wherein at least one said helical coil is formed to interlock into an abutting relationship with adjacent said helical coils.

3. The overtube of claim 2 wherein said at least one said helical coil has a protrusion (620) formed on one side thereof and a pocket (622) formed on another side thereof.

4. The overtube of claim 1 wherein said substantially flexible helically wound continuous member has a cross-sectional shape in the form of a parallelogram.

5. The overtube of claim 4 wherein said substantially flexible helically wound continuous member is fabricated from metal material.

6. A surgical kit comprising:
an endoscope (10); and
an overtube (100) of claim 1.

7. The overtube of claim 2 wherein said flexible helically wound continuous member is wound around a substantially flexible tubular member.

8. The overtube of claim 7 wherein said flexible helically wound continuous member is integrally formed with said flexible tubular member.

9. A method for processing an instrument for surgery, the method comprising:
obtaining the overtube of claim 1 or 2;
sterilizing the overtube; and
storing the overtube in a sterile container.

## Patentansprüche

1. Außenröhre (100) zur Verwendung mit einem chirurgischen Endoskopieinstrument (10), wobei die Außenröhre umfasst:
ein im Wesentlichen flexibles spiralförmig gewickeltes kontinuierliches Element (110), eine Reihe von Spiralspulen (112) bildend, die einen hohlen Durchgang bilden, bemessen zur Aufnahme eines Teils (14) des chirurgischen Endoskopieinstruments dort hindurch, wobei die Außenröhre ein distales Ende und ein proximales Ende aufweist; und
mindestens ein versteifendes Element, gekoppelt mit der Außenröhre, zum selektiven Ziehen der Spiralspulen in anstoßenden Kontakt miteinander, um die Außenröhre zu versteifen, wobei das mindestens eine versteifende Element umfasst:
eine Mehrzahl von spannenden Elementen (232), gekoppelt mit der Außenröhre und dort hindurch verlaufend, und
einen Aktor (320), wirkverbunden mit der Mehrzahl von spannenden Elementen;
**dadurch gekennzeichnet, dass** die Spiralspulen mit Lumina (130) zur Aufnahme der Mehrzahl von spannenden Elementen dort hindurch bereitgestellt sind.

2. Außenröhre nach Anspruch 1, wobei mindestens eine Spiralspule ausgebildet ist zum Verriegeln in einer anstoßenden Beziehung mit den benachbarten Spiralspulen.

3. Außenröhre nach Anspruch 2, wobei die mindestens eine Spiralspule einen an einer Seite davon ausgebildeten Vorsprung (620) und eine an einer anderen Seite davon ausgebildete Tasche (622) aufweist.

4. Außenröhre nach Anspruch 1, wobei das im Wesentlichen flexible spiralförmig gewickelte kontinuierliche Element eine Querschnittsform in Form eines Parallelogramms aufweist.

5. Außenröhre nach Anspruch 4, wobei das im Wesentlichen flexible spiralförmig gewickelte kontinuierliche Element aus Metallmaterial hergestellt ist.

6. Chirurgisches Kit, umfassend:
ein Endoskop (10); und
eine Außenröhre (100) nach Anspruch 1.

7. Außenröhre nach Anspruch 2, wobei das flexible spiralförmig gewickelte kontinuierliche Element um ein im Wesentlichen flexibles rohrförmiges Element gewickelt ist.

8. Außenröhre nach Anspruch 7, wobei das flexible spiralförmig gewickelte kontinuierliche Element in einem Stück mit dem flexiblen rohrförmigen Element ausgebildet ist.

9. Verfahren zum Verarbeiten eines Instruments für die Chirurgie, wobei das Verfahren umfasst:
Erhalten der Außenröhre nach Anspruch 1 oder 2;
Sterilisieren der Außenröhre; und
Lagern der Außenröhre in einem sterilen Behälter.

## Revendications

1. Surtube (100) à utiliser avec un instrument chirurgical endoscopique (10), ledit surtube comprenant :
un élément continu enroulé hélicoïdalement sensiblement flexible (110) formant une série de bobines hélicoïdales (112) qui définissent un passage creux dimensionné pour y recevoir une partie (14) de l'instrument chirurgical endoscopique, ledit surtube ayant une extrémité distale et une extrémité proximale ; et
au moins un élément raidisseur couplé audit surtube pour tirer sélectivement lesdites bobines hélicoïdales en contact de butée les unes avec les autres pour rigidifier ledit surtube, ledit au moins un élément raidisseur comprenant :
une pluralité d'éléments de tension (232) couplés audit surtube s'étendant à travers celui-ci ; et
un actionneur (320) fonctionnellement couplé à ladite pluralité d'éléments de tension ;
**caractérisé en ce que** les bobines hélicoïdales sont pourvues de lumières (130) destinées à y recevoir ladite pluralité d'éléments de tension.

2. Surtube de la revendication 1 dans lequel au moins une dite bobine hélicoïdale est formée pour se verrouiller en relation de butée avec lesdites bobines hélicoïdales adjacentes.

3. Surtube de la revendication 2 dans lequel ladite au moins une dite bobine hélicoïdale a une saillie (620) formée sur un côté de celle-ci et une poche (622) formée sur un autre côté de celle-ci.

4. Surtube de la revendication 1 dans lequel ledit élément continu enroulé hélicoïdalement sensiblement flexible a une forme en coupe transversale sous la forme d'un parallélogramme.

5. Surtube de la revendication 4 dans lequel ledit élément continu enroulé hélicoïdalement sensiblement flexible est fabriqué à partir d'un matériau métallique.

6. Trousse chirurgicale comprenant :
un endoscope (10) ; et
un surtube (100) de la revendication 1.

7. Surtube de la revendication 2 dans lequel ledit élément continu enroulé hélicoïdalement flexible est enroulé autour d'un élément tubulaire sensiblement flexible.

8. Surtube de la revendication 7 dans lequel ledit élément continu enroulé hélicoïdalement flexible est formé d'une seule pièce avec ledit élément tubulaire flexible.

9. Procédé de traitement d'un instrument de chirurgie, le procédé comprenant :
l'obtention du surtube de la revendication 1 ou 2 ;
la stérilisation du surtube ; et
le stockage de surtube dans un récipient stérile.
